# EUROPEAN PATENT APPLICATION

(11) **EP 4 306 949 A1**
(43) Date of publication of application: **17.01.2024**
(21) Application number: 22767117.9
(22) Date of filing: 08.03.2022
(51) Int. Cl.: G01N 27/416, C01B 13/11

(54) **ELECTROCHEMICAL GAS SENSOR, OZONE GENERATOR, AND HUMIDIFIER**

(30) Priority: 08.03.2021 JP 2021035918; 07.09.2021 JP 2021145229; 22.12.2021 JP 2021208078
(71) Applicant: Niterra Co., Ltd., Nagoya-shi, Aichi 461-0005 (JP)
(72) Inventor: KITO, Shinichiro, Nagoya-shi, Aichi 461-0005 (JP); SEGAWA, Masayuki, Nagoya-shi, Aichi 461-0005 (JP); YOKOYAMA, Takahiro, Nagoya-shi, Aichi 461-0005 (JP); IMAIZUMI, Junya, Nagoya-shi, Aichi 461-0005 (JP); MIZUTANI, Yuki, Nagoya-shi, Aichi 461-0005 (JP); TANAKA, Masahiro, Nagoya-shi, Aichi 461-0005 (JP); KUMAGAI, Yoshiko, Nagoya-shi, Aichi 461-0005 (JP)
(74) Representative: Grünecker Patent- und Rechtsanwälte PartG mbB
(86) International application number: PCT/JP2022/009861
(87) International publication number: WO 2022/191163

(57) **Abstract**

In an electrochemical gas sensor (10), a first sensing element (21) is stored in a first storage portion (31). A moisture permeable film (24) is disposed in a first introduction inlet (31A) of the first storage portion (31). The moisture permeable film (24) substantially prevents a to-be-detected gas from permeating therethrough. A second sensing element (22) is disposed in a space into which water vapor and the to-be-detected gas contained in a target gas flow. In such a configuration, the electrochemical gas sensor (10) is capable of detecting a to-be-detected gas having a concentration of 0 or more and 1 ppm or less.

## Description

### TECHNICAL FIELD

The present invention relates to an electrochemical gas sensor, an ozone generator, and a humidifier.

### BACKGROUND ART

Patent Document 1 discloses an example of an electrochemical gas sensor. The ozone sensor disclosed in Patent Document 1 is an electrochemical sensor in which diaphragm polarography is used, and quantifies ozone gas according to a magnitude of an electric current flowing between both electrodes: a working electrode and a counter electrode.

Patent Document 2 discloses an example of an ozone generation apparatus. The ozone generation apparatus performs feedback control based on a signal from an external ozone sensor such that an external ozone concentration in a space outside the ozone generation apparatus becomes a target external ozone concentration.

### PRIOR ART DOCUMENT

### PATENT DOCUMENT

Patent Document 1: Japanese Patent Application Laid-Open (kokai) No. 2020-16593
Patent Document 2: Japanese Patent Application Laid-Open (kokai) No. 2019-99398

### SUMMARY OF THE INVENTION

### PROBLEM TO BE SOLVED BY THE INVENTION

When a concentration of a to-be-detected gas contained in gas is detected by an electrochemical gas sensor, an error due to humidity may occur under an environment in which humidity varies. Particularly in a case where a concentration of a to-be-detected gas contained in gas is low, influence of humidity becomes relatively great, so that error is more likely to occur.

The present invention has been made in order to solve at least one of the above-described problems, and one of the objects is to provide a technique capable of more correctly detecting a concentration of a to-be-detected gas even when the concentration of the to-be-detected gas contained in a target gas is low.

### MEANS FOR SOLVING THE PROBLEM

An electrochemical gas sensor according to the present invention is directed to a gas sensor for detecting a concentration of a to-be-detected gas contained in a target gas, and the electrochemical gas sensor includes:
a first sensing element configured to electrochemically detect a concentration of gas;
a second sensing element disposed in a space into which water vapor and the to-be-detected gas contained in the target gas flow and configured to electrochemically detect a concentration of gas;
a first storage portion having, in its own structure, an internal space in which the first sensing element is stored;
a first introduction inlet provided between the internal space and an external space outside the first storage portion; and
a moisture permeable film disposed in the first introduction inlet and configured to substantially prevent permeation of the to-be-detected gas from the external space to the internal space.

The electrochemical gas sensor is capable of cancelling influence of water vapor based on an output of the first sensing element and an output of the second sensing element and detecting the to-be-detected gas having a concentration of 0 or more and 1 ppm or less (parts per million).

In an electrochemical gas sensor, when there is a difference in an amount of water vapor between inside and outside of the element, an electromotive force caused by the difference in the amount of water vapor may be generated, and an electric current caused by the difference in the amount of water vapor may flow. In the case of a low-concentration target gas in which a concentration of a to-be-detected gas is 1 ppm or less, influence of an electric current caused by difference in an amount of water vapor tends to be great when detecting the concentration of the to-be-detected gas. Regarding this, in the above-described electrochemical gas sensor, since the first sensing element detects a concentration of a to-be-detected gas in a state in which the to-be-detected gas is substantially removed from a target gas, influence of an electric current caused by difference in an amount of water vapor can be evaluated. Meanwhile, the second sensing element can detect a concentration of a target component containing water vapor and the to-be-detected gas. That is, the electrochemical gas sensor can use the detection result of the first sensing element (result of evaluating influence of an electric current caused by difference in an amount of water vapor) for detecting a concentration of the to-be-detected gas by the second sensing element, which is advantageous in correctly detecting a concentration of a to-be-detected gas in a target gas in which the concentration of the to-be-detected gas is low. Therefore, even when a to-be-detected gas contained in a target gas has a low concentration of 1 ppm or less, the above-described electrochemical gas sensor can more correctly detect a concentration of the to-be-detected gas.

In the description herein, "substantially prevent permeation of the to-be-detected gas" means that a permeation amount of the to-be-detected gas is 1/50 or less of a permeation amount of water vapor in terms of volume.

The moisture permeable film may be a water vapor permeable filter that allows permeation of water vapor from the external space to the internal space and substantially prevents permeation of the to-be-detected gas from the external space to the internal space.

In the electrochemical gas sensor, a function of allowing permeation of water vapor from the external space to the internal space and substantially preventing permeation of the to-be-detected gas from the external space to the internal space can be achieved by the water vapor permeable filter.

In the electrochemical gas sensor, a thickness of the moisture permeable film may be more than 0 um and 60 um or less.

When the moisture permeable film is structured to have a thickness of 60 um or less, degradation of responsiveness with respect to change of a humidity can be further inhibited in the detection by the first sensing element.

The electrochemical gas sensor may be preferably capable of detecting the to-be-detected gas having a concentration of 0 or more and 100 ppb or less (parts per billion).

In the case of an extremely-low-concentration target gas in which a concentration of the to-be-detected gas is 100 ppb or less, influence of an "electric current caused by difference in an amount of water vapor" described above tends to be much greater when detecting the concentration of the to-be-detected gas. Regarding this, the electrochemical gas sensor can correctly evaluate influence of an "electric current caused by difference in an amount of water vapor" and detect a concentration of the to-be-detected gas by reflecting the evaluation result, so that the electrochemical gas sensor is very useful in a case where a target is gas in which the concentration of the to-be-detected gas is 100 ppb or less.

The electrochemical gas sensor may include: a sensitivity setting portion configured to set a sensitivity based on a humidity of the target gas detected by a humidity sensor; and a concentration specifying portion configured to specify a concentration of the to-be-detected gas based on an output of the first sensing element, an output of the second sensing element, and the sensitivity.

If humidity is changed, an "appropriate sensitivity" may be changed. However, the above-described electrochemical gas sensor can adjust sensitivity according to change of humidity. The humidity sensor may be configured as a part of the electrochemical gas sensor or may be a humidity sensor different from the electrochemical gas sensor.

In the electrochemical gas sensor, the to-be-detected gas may be ozone.

When a concentration of ozone is detected by the gas sensor, water vapor may become noise, and the ozone concentration may be hindered from being correctly detected. Particularly, when a target is gas in which a concentration of ozone is low, influence of an "electric current caused by difference in an amount of water vapor" described above becomes relatively great, so that such a problem becomes more significant. However, the above-described electrochemical gas sensor can correctly evaluate influence of an "electric current caused by difference in an amount of water vapor", and can use the evaluation for detecting a concentration of ozone. Therefore, an ozone concentration is likely to be correctly detected even in a target gas in which an ozone concentration is low.

An ozone generator may include the above-described electrochemical gas sensor in which the to-be-detected gas is ozone.

The ozone generator can correctly detect a concentration of ozone in a simple configuration in addition to merely generating ozone.

A humidifier may include the above-described electrochemical gas sensor.

The humidifier can correctly detect a concentration of a to-be-detected gas in a simple configuration in addition to merely performing humidification.

In a case where the ozone generator includes the above-described electrochemical gas sensor, the ozone generator may include an ozone supply portion, a sensing portion, a controller, and an abnormality determination portion. In this configuration, the ozone supply portion may be configured to supply ozone to the external space. The sensing portion may be configured to electrochemically detect an ozone concentration, and may have the first sensing element and the second sensing element. The controller may be configured to perform first control for performing feedback control for the ozone supply portion such that an ozone concentration detected by the sensing portion approaches a target concentration, and second control for stopping the first control in a case where a determination criterion is satisfied during the first control. Furthermore, the abnormality determination portion may determine abnormality based on a degree of change from satisfaction of the determination criterion of an ozone concentration detected by the sensing portion until elapse of a predetermined period.

In the ozone generator, when the first control is stopped, a degree of change of an ozone concentration detected by the sensing portion (detected concentration) varies depending on an actual ozone concentration. Therefore, based on the degree of change, the ozone generator can determine abnormality in which an actual ozone concentration during feedback control deviates from the target concentration.

In the electrochemical gas sensor disclosed in Patent Document 2, not only ozone gas but also gas other than ozone gas may exert an influence on a magnitude of an electric current flowing between both a working electrode and a counter electrode. As a result, an ozone concentration measured by a sensor may deviate from an actual external ozone concentration. Therefore, for example, in a case where feedback control disclosed in Patent Document 1 is performed by using the electrochemical gas sensor disclosed in Patent Document 2, an actual external ozone concentration may deviate from a target external ozone concentration. Regarding this, the above-described ozone generator can determine abnormality in which an actual ozone concentration during feedback control deviates from a target concentration.

In the second control, the controller may control the ozone supply portion so as to supply low-concentration ozone or stop the ozone supply portion. The abnormality determination portion may determine abnormality based on a degree of reduction from satisfaction of the determination criterion of an ozone concentration detected by the sensing portion until elapse of the predetermined period.

In this configuration, abnormality can be determined while inhibiting increase of the ozone concentration in the external space.

In the ozone generator, the abnormality determination portion may determine abnormality in a case where the degree of reduction is determined to be greater than a threshold value.

In this configuration, abnormality in which an actual ozone concentration during feedback control becomes higher than a target concentration, can be determined.

In the ozone generator, the controller may perform the first control in a case where the degree of reduction is determined to be not greater than the threshold value, and may perform third control in a case where the degree of reduction is determined to be greater than the threshold value. In the third control, the controller may perform feedback control for the ozone supply portion such that a concentration detected by the sensing portion approaches a second target concentration that is lower than the target concentration, or cause the ozone supply portion so as to supply low-concentration ozone, or stop the ozone supply portion is stopped.

In this configuration, in a case where the degree of reduction is determined to be not greater than a threshold value, the first control is restarted, and, in a case where the degree of reduction is determined to be greater than the threshold value, supply of ozone can be reduced or stopped.

In the ozone generator, when the controller causes the ozone supply portion so as to supply low-concentration ozone in the third control, the controller may control the ozone supply portion such that the greater the degree of reduction is, the less a supply amount of ozone is.

In this configuration, the higher the actual ozone concentration is assumed to be, the less the supply amount of ozone can be made.

The ozone generator may include a humidifying portion for humidifying the external space.

In this configuration, the ozone generator can also function as a humidifier.

### ADVANTAGEOUS EFFECTS OF THE INVENTION

According to the present invention, even when a concentration of a to-be-detected gas contained in a target gas is low, the concentration of the to-be-detected gas can be more correctly detected.

### BRIEF DESCRIPTION OF THE DRAWINGS

[Fig. 1] Fig. 1 is a circuit diagram illustrating an electric configuration of an electrochemical gas sensor according to a first embodiment in a simplified manner.
[Fig. 2] Fig. 2 is a schematic cross-sectional view illustrating an element portion of the electrochemical gas sensor according to the first embodiment.
[Fig. 3] Fig. 3 is an explanatory diagram conceptually illustrating a sensitivity and sensitivity correction in the electrochemical gas sensor according to the first embodiment.
[Fig. 4] Fig. 4 is a graph showing an experimental result of Experiment 1.
[Fig. 5] Fig. 5 is a graph showing an experimental result of Experiment 2.
[Fig. 6] Fig. 6 is an explanatory diagram illustrating an ozone generator including the electrochemical gas sensor according to the first embodiment.
[Fig. 7] Fig. 7 is a block diagram schematically illustrating an ozone generator according to a second embodiment.
[Fig. 8] Fig. 8 is a graph showing ozone concentrations changing with elapse of time from stop of first control.
[Fig. 9] Fig. 9 is a flowchart showing a flow of a process performed by the ozone generator.

### MODES FOR CARRYING OUT THE INVENTION

### 1. First embodiment

### 1-1. Configuration of electrochemical gas sensor

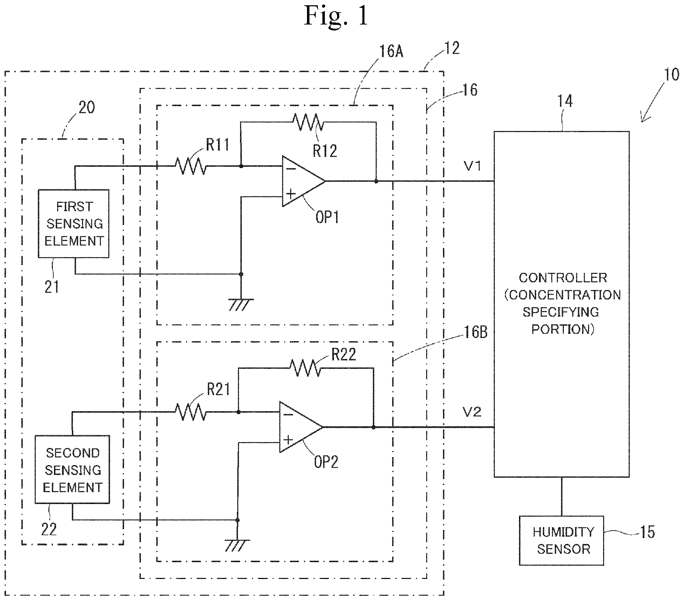

An electrochemical gas sensor 10 illustrated in Fig. 1 is a gas sensor for detecting a concentration of a to-be-detected gas contained in a target gas. The electrochemical gas sensor 10 is simply referred to also as a gas sensor 10. In a typical example of the electrochemical gas sensor 10 described below, the to-be-detected gas is ozone (ozone gas). That is, the gas sensor 10 illustrated in Fig. 1 functions as an ozone gas sensor for detecting a concentration of ozone gas. The gas sensor 10 mainly includes a sensing portion 12 and a controller 14. The sensing portion 12 includes an element portion 20 and an output circuit portion 16.

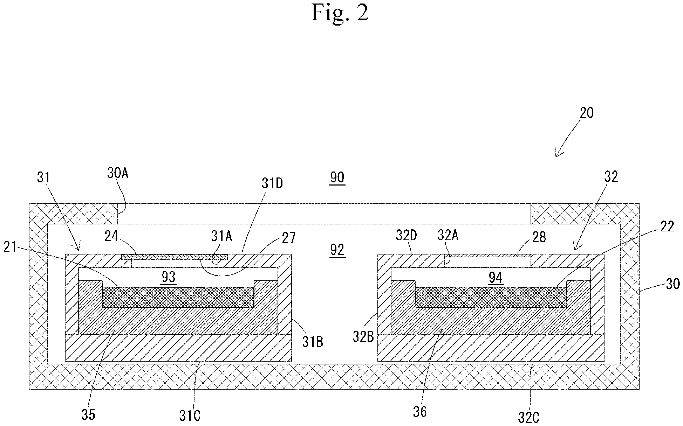

The element portion 20 generates a first signal (voltage V1) as a signal for evaluating an "electric current caused by difference in an amount of water vapor" described above, and a second signal (voltage V2) as a signal corresponding to a concentration of a to-be-detected gas contained in the target gas. As illustrated in Fig. 2, the element portion 20 includes a first sensing element 21, a second sensing element 22, a first storage portion 31, a second storage portion 32, a first introduction inlet 31A, a second introduction inlet 32A, a moisture permeable film 24, a first waterproof filter 27, a second waterproof filter 28, and a case 30.

The case 30 is a storage portion that stores the first storage portion 31, the second storage portion 32, the first sensing element 21, the second sensing element 22, and the like. The case 30 has an opening 30A that connects between a space 90 outside the case 30 and a space 92 inside the case 30. Fig. 2 illustrates an example of the opening 30A. However, the structure of the opening 30A is not limited as long as gas present in the space 90 outside the case 30 can be introduced into the space 92 inside the case 30. In the example illustrated in Fig. 2, the first introduction inlet 31A and the second introduction inlet 32A are provided in the case 30. However, the first introduction inlet 31A and the second introduction inlet 32A may be provided in the space 90 outside the case 30. That is, a structure in which gas directly enters the moisture permeable film 24, the first waterproof filter 27, and the second waterproof filter 28 from the space 90, may be employed.

In the first embodiment, the "target gas" containing a to-be-detected gas of which the concentration is to be detected by the gas sensor 10 is gas that is present in the space 90 outside the case 30 and that enters the space 92 inside the case 30 through the opening 30A formed in the case 30. In the example illustrated in Fig. 2, gas in the spaces 90 and 92 corresponds to an example of the "target gas". The spaces 90 and 92 each correspond to an example of an external space outside the first storage portion 31.

In the case 30, the first storage portion 31 and the second storage portion 32 are disposed. The first storage portion 31 stores the first sensing element 21 thereinside. The first storage portion 31 has, in its own structure, an internal space 93 in which the first sensing element 21 is stored. The first storage portion 31 is in the form of a box that has a side wall portion 31B that surrounds the peripheral portion of the first sensing element 21, a bottom wall portion 31C that closes the lower side of the first sensing element 21, and an opening (the first introduction inlet 31A) formed in an upper portion. Specifically, an upper wall portion 31D and the bottom wall portion 31C oppose each other in the up-down direction, the upper wall portion 31D is connected to the upper end portion of the side wall portion 31B, the bottom wall portion 31C is connected to the lower end portion of the side wall portion 31B, and the upper wall portion 31D has the above-described opening (first introduction inlet 31A) formed therein. In the example illustrated in Fig. 2, the thickness direction of the moisture permeable film 24 described below is the up-down direction. In the first storage portion 31, the upper wall portion 31D side (side on which the first introduction inlet 31A is formed) is the upper side, and the bottom wall portion 31C side is the lower side.

The first introduction inlet 31A is an opening formed in the first storage portion 31. The first introduction inlet 31A penetrates, in the up-down direction, the upper wall portion 31D forming a part of the first storage portion 31.

The moisture permeable film 24 is a filter disposed in the first introduction inlet 31A. The moisture permeable film 24 is a film body that has a function of taking moisture in the external space (spaces 90, 92) thereinto (into the film), and guiding, to the internal space 93, moisture corresponding to the taken moisture, and that substantially does not allow permeation of a to-be-detected gas. For example, the moisture permeable film 24 may be configured to take in moisture in the external space (spaces 90, 92), cause the moisture to pass through the moisture permeable film 24, and guide the moisture to the internal space 93. Alternatively, the moisture permeable film 24 may be configured to take in moisture in the external space (spaces 90, 92), perform ion exchange thereinside, and guide moisture generated by the ion exchange to the internal space 93. For the moisture permeable film 24, polystyrene sulfonate, polyvinyl alcohol, vinyl alcohol copolymers, a fluorine-based ion exchange resin, a resin having a protic hydrophilic group in a repeating unit, a resin having an aprotic hydrophilic group in a repeating unit, or the like can be used. Examples of the fluororesin-based ion exchange membrane include Nafion (registered trademark), Flemion (registered trademark), and Aciplex (registered trademark). The moisture permeable film 24 may be used overlapping with a hydrophobic porous membrane. That is, any configuration may be used as long as an absolute humidity in the internal space 93 and an absolute humidity in the external space 92 can be made approximately equal to each other by presence of the moisture permeable film 24 disposed in the first introduction inlet 31A.

In the typical example of the present embodiment, the moisture permeable film 24 is formed by, for example, a water vapor permeable filter, and allows permeation of water vapor from the external space (spaces 90, 92) to the internal space 93, and substantially prevents permeation of ozone (ozone gas) from the external space (spaces 90, 92) to the internal space 93. In the moisture permeable film 24, a permeation amount of ozone gas is 1/50 or less of a permeation amount of water vapor in terms of volume. The moisture permeable film 24 is provided so as to close the first introduction inlet 31A. Therefore, gas entering the internal space 93 from the external space (spaces 90, 92) passes through the moisture permeable film 24 and enters the internal space 93.

The thickness of the moisture permeable film 24 is preferably more than 0 um and 60 um or less. When the thickness of the moisture permeable film 24 is 60 um or less, the moisture permeable film 24 is advantageous in responsiveness. The thickness of the moisture permeable film 24 may be more preferably 30 um or less. When the thickness of the moisture permeable film 24 is 30 um or less, the moisture permeable film 24 is more advantageous in responsiveness. Meanwhile, the thickness of the moisture permeable film 24 is more preferably 1 um or more. When the thickness of the moisture permeable film 24 is 1 um or more, the moisture permeable film 24 is advantageous from the viewpoint of production. The thickness of the moisture permeable film 24 may be more preferably 5 um or more. When the thickness of the moisture permeable film 24 is 5 um or more, the moisture permeable film 24 is more advantageous in production. For example, when the thickness of the moisture permeable film 24 is 1 um or more and 30 um or less, the moisture permeable film 24 is advantageous in production and responsiveness in a well-balanced manner.

The first waterproof filter 27 is a filter that allows gas such as water vapor and ozone to pass therethrough, and substantially prevents liquid from passing therethrough. In the first waterproof filter 27, a permeation amount of liquid is 1/50 or less of a permeation amount of gas in terms of volume. The first waterproof filter 27 is provided so as to close the first introduction inlet 31A. Therefore, gas entering the internal space 93 from the external space (spaces 90, 92) passes through the first waterproof filter 27 and enters the internal space 93.

The second storage portion 32 stores the second sensing element 22 thereinside. The second storage portion 32 has, in its own structure, an internal space 94 in which the second sensing element 22 is stored. The second storage portion 32 is in the form of a box that has a side wall portion 32B that surrounds the peripheral portion of the second sensing element 22, a bottom wall portion 32C that closes the lower side of the second sensing element 22, and an opening (the second introduction inlet 32A) formed in an upper portion. Specifically, an upper wall portion 32D and the bottom wall portion 32C oppose each other in the up-down direction, the upper wall portion 32D is connected to the upper end portion of the side wall portion 32B, the bottom wall portion 32C is connected to the lower end portion of the side wall portion 32B, and the upper wall portion 32D has the above-described opening (second introduction inlet 32A) formed therein. In the second storage portion 32, the upper wall portion 32D side (side on which the second introduction inlet 32A is formed) is the upper side, and the bottom wall portion 32C side is the lower side.

The second introduction inlet 32A is an opening formed in the second storage portion 32. The second introduction inlet 32A is a flow path that can function to introduce gas. The second introduction inlet 32A penetrates, in the up-down direction, the upper wall portion 32D forming a part of the second storage portion 32. The second introduction inlet 32A forms a route in which gas passes between the external space (space 90 or the space 92) outside the second storage portion 32 and the internal space 94 inside the second storage portion 32.

The second waterproof filter 28 is a filter that allows gas such as water vapor and ozone to pass therethrough, and substantially prevents liquid from passing therethrough. In the second waterproof filter 28, a permeation amount of liquid is 1/50 or less of a permeation amount of gas in terms of volume. The second waterproof filter 28 is provided so as to close the second introduction inlet 32A. Therefore, gas entering the internal space 94 from the external space (spaces 90, 92) passes through the second waterproof filter 28 and enters the internal space 94.

Each of the first sensing element 21 and the second sensing element 22 is an element that electrochemically causes an electric current corresponding to a concentration of ozone (ozone gas) to flow therethrough. However, each of the first sensing element 21 and the second sensing element 22 is also an element that generates an electromotive force corresponding to difference between an amount of water vapor inside the element and an amount of water vapor outside the element. The first sensing element 21 and the second sensing element 22 have the same configuration. In each of the first sensing element 21 and the second sensing element 22, when ozone gas is present in a space therearound, a reduction reaction occurs in its own detection electrode (not illustrated) and an oxidation reaction occurs in its own counter electrode (not illustrated). Therefore, an electric current corresponding to an ozone gas concentration flows due to such reactions. However, in addition to an electric current corresponding to an ozone gas concentration, each of the first sensing element 21 and the second sensing element 22 also causes flow of an "electric current caused by difference in an amount of water vapor". Specifically, in the first sensing element 21 and the second sensing element 22, when there is a difference between an amount of water vapor in the gas flowing in from the space 92 and an amount of water vapor inside the element (space inside the element or electrolyte inside the element), an electric current corresponding to the difference is generated.

For example, in the first sensing element 21, an electric current corresponding to an ozone gas concentration in the internal space 93 and an electric current caused by difference in an amount of water vapor between inside and outside the first sensing element 21 flow between a detection electrode and a counter electrode thereof. Specifically, the above-described reaction occurs in an electrolyte in the first sensing element 21 due to the ozone gas supplied to the first sensing element 21 via the internal space 93, and thereby an electric current corresponding to an ozone gas concentration flows, and, in addition to the electric current, an electric current caused by difference in an amount of water vapor between inside and outside the first sensing element 21 flows. The relationship between an ozone gas concentration in the internal space 93 and an "electric current corresponding to an ozone gas concentration" generated in the first sensing element 21 is specified by a predetermined arithmetic expression, and the higher an ozone gas concentration in the internal space 93 is, the higher an "electric current corresponding to the ozone gas concentration" generated in the first sensing element 21 is. However, flow of ozone gas into the internal space 93 is substantially prevented by the moisture permeable film 24. Therefore, in the first sensing element 21, an electric current corresponding to an ozone gas concentration does not flow, or even if the electric current flows, it is extremely low.

Similarly, in the second sensing element 22, an electric current corresponding to an ozone gas concentration in the internal space 94 and an electric current caused by difference in an amount of water vapor between inside and outside the second sensing element 22 flow between a detection electrode and a counter electrode thereof. Specifically, the above-described reaction occurs in an electrolyte in the second sensing element 22 due to the ozone gas supplied to the second sensing element 22 via the internal space 94, and thereby an electric current corresponding to an ozone gas concentration flows, and, in addition to the electric current, an electric current caused by difference in an amount of water vapor between inside and outside the second sensing element 22 flows. The relationship between an ozone gas concentration in the internal space 94 and an "electric current corresponding to an ozone gas concentration" generated in the second sensing element 22 is specified by a predetermined arithmetic expression, and the higher an ozone gas concentration in the internal space 94 is, the higher an "electric current corresponding to the ozone gas concentration" generated in the second sensing element 22 is. In a case where difference in an amount of water vapor between inside and outside the first sensing element 21 and difference in an amount of water vapor between inside and outside the second sensing element 22 are approximately equal to each other, an "electric current caused by difference in an amount of water vapor" generated in the second sensing element 22 and an "electric current caused by difference in an amount of water vapor" generated in the first sensing element 21 are approximately equal to each other.

The output circuit portion 16 includes a first output circuit 16A and a second output circuit 16B. The first output circuit 16A has resistances R11 and R12 and an operational amplifier OP1. Ra represents a resistance value of the resistance R11. Rb represents a resistance value of the resistance R12. The first output circuit 16A is a circuit that converts an electric current Ia (hereinafter, also referred to as output Ia) generated in the first sensing element 21 to a voltage Va, and outputs a voltage V1 obtained by amplifying the voltage Va at a predetermined amplification factor (Rb/Ra). In the first output circuit 16A, the electric current Ia generated in the first sensing element 21 flows through the resistance R11, and the voltage Va (Va=Ia×Ra) corresponding to the electric current Ia is generated between both ends of the resistance R11. A relationship among the voltage Va, the voltage V1 outputted by the first output circuit 16A to the controller 14, and the electric current Ia generated in the first sensing element 21 satisfies V1=Va×Rb/Ra=Ia×Rb.

Similarly, the second output circuit 16B has resistances R21 and R22 and an operational amplifier OP2. Rc represents a resistance value of the resistance R21. Rd represents a resistance value of the resistance R22. The second output circuit 16B is a circuit that converts an electric current Ib (hereinafter, also referred to as output Ib) generated in the second sensing element 22 to a voltage Vb, and outputs a voltage V2 obtained by amplifying the voltage Vb at a predetermined amplification factor (Rd/Rc). In the second output circuit 16B, the electric current Ib generated in the second sensing element 22 flows through the resistance R21, and the voltage Vb(Vb=Ib×Rc) corresponding to the electric current Ib is generated between both ends of the resistance R21. A relationship among the voltage Vb, the voltage V2 outputted by the second output circuit 16B to the controller 14, and the electric current Ib generated in the second sensing element 22 satisfies V2=Vb×Rd/Rc=Ib×Rd.

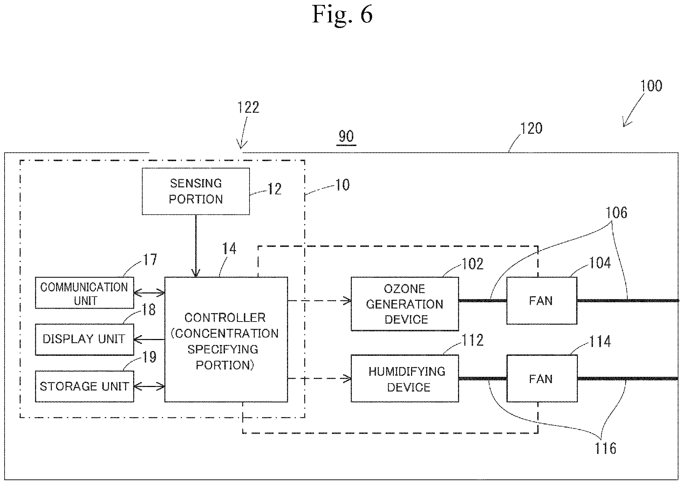

A humidity sensor 15 is configured as, for example, an absolute humidity sensor that has a known configuration and detects an absolute humidity in the space 90. In Fig. 6, the humidity sensor 15 is not illustrated. The humidity sensor 15 may be configured as a relative humidity sensor. The humidity sensor 15 may not necessarily be provided.

The controller 14 illustrated in Fig. 1 is configured as an information processing device having a function of performing an arithmetic operation, an information processing function, a control function, and the like. The controller 14 includes an AD converter, an MCU (micro controller unit), and the like. To the controller 14, the voltage V1 is inputted from the first output circuit 16A, and the voltage V2 is inputted from the second output circuit 16B. The controller 14 can convert the voltages V1 and V2 to digital signals, and can perform an arithmetic operation using the voltages V1 and V2.

### 1-2. Method for calculating ozone gas concentration

In the gas sensor 10 of the present embodiment, for example, Ra=Rc and Rb=Rd are satisfied, and an amplification factor at the first output circuit 16A and an amplification factor at the second output circuit 16B are equal to each other. The first sensing element 21 and the second sensing element 22 are the same. In the first sensing element 21, an electric current caused by difference in an amount of water vapor (electric current corresponding to difference in an amount of water vapor between inside and outside the first sensing element 21) flows. In the second sensing element 22, in addition to an electric current caused by difference in an amount of water vapor (electric current corresponding to difference in an amount of water vapor between inside and outside the second sensing element 22), an electric current corresponding to an ozone concentration in the external space 92 is superimposed and flows. Therefore, the value of V2-V1 is a value obtained by cancelling influence of an "electric current caused by difference in an amount of water vapor" generated in the first sensing element 21 and the second sensing element 22, and is a voltage value corresponding to an ozone gas concentration in the space 92.

In the sensing portion 12, the value of V2-V1 and an ozone gas concentration Y1 have such a correlation that the higher the ozone gas concentration Y1 in the space 92 is, the greater the value of V2-V1 is. The correlation can be represented by, for example, a relational expression (linear expression) of Y1=β(V2-V1)+B. In the relational expression, the value of B is a predetermined fixed value (constant). The value of β may be a predetermined fixed value (constant), but may be a variable which is determined based on an absolute humidity. That is, the value of the gradient β in the relational expression (linear expression) may be corrected according to an absolute humidity.

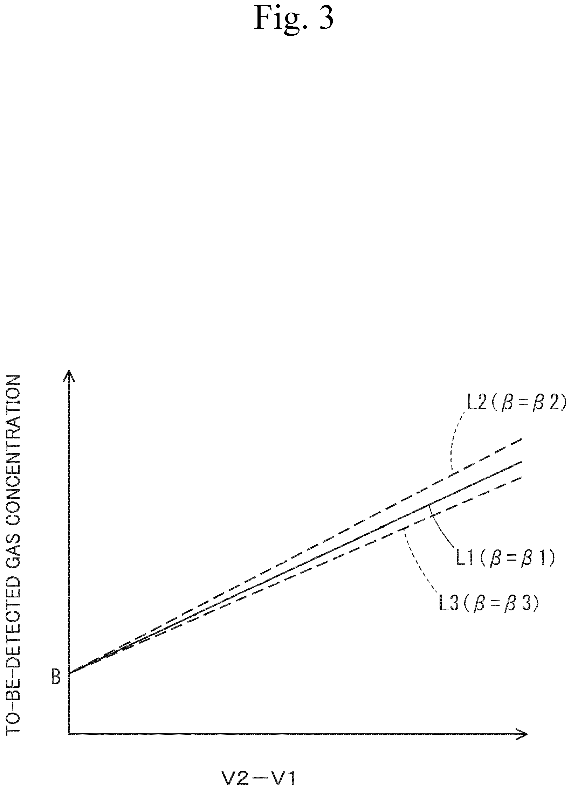

In a typical example described below, β is obtained according to a correction expression of β=m×α+n. In the correction expression, each of m and n represents a predetermined fixed value. α represents an absolute humidity of a target gas. Specifically, for example, α represents an absolute humidity detected in the spaces 90 and 92 by the humidity sensor 15. β corresponds to a gradient of the above-described relational expression (Y1=β(V2-V1)+B). Therefore, the relationship between Y1 and V2-V1 can be corrected by adjusting the value of β. For example, in a case where the relational expression when the value of β is β1 is as represented by a linear line L1 illustrated in Fig. 3, the relational expression can be adjusted as linear lines L2 and L3 by adjusting the value of β to β2 and β3.

In the above-described example, the value of β corresponds to an example of a sensitivity. The controller 14 corresponds to an example of a sensitivity setting portion, and sets a sensitivity β based on an absolute humidity α of a target gas detected by the humidity sensor 15. The controller 14 corresponds to an example of a concentration characteristic portion, and calculates the concentration Y1 of ozone gas (to-be-detected gas), according to the above-described relational expression and correction expression, based on the output Ia of the first sensing element 21, the output Ib of the second sensing element 22, and the sensitivity β.

The calculated concentration Y1 may be adopted as the ozone gas concentration as it is, but it is desirable to apply a moving average assuming that delay in response may occur. For example, in a case where the ozone gas concentration Y1 is repeatedly detected cyclically at predetermined time intervals, a simple moving average may be calculated so as to obtain an average of the most recent n pieces of data (the concentrations Y1), and the value may be adopted as an ozone gas concentration.

In this way, the gas sensor 10 can cancel influence of water vapor based on the output Ia of the first sensing element 21 and the output Ib of the second sensing element 22 to obtain the ozone gas concentration Y1. Specifically, as described above, the value of V2-V1 is determined according to the expression of V1=Rb×Ia and the expression of V2=Rd×Ib=Rb×Ib. As described above, the value of V2-V1 is a value obtained by canceling influence of an "electric current caused by difference in an amount of water vapor" generated in the first sensing element 21 and the second sensing element 22, is a voltage value corresponding to an ozone gas concentration in the space 92, and is a value represented by the expression of Rb(Ib-Ia). That is, the gas sensor 10 can cancel influence of water vapor based on difference (Ib-Ia) between the output Ia and the output Ib to detect an ozone gas concentration, and furthermore, can detect ozone gas that has a low concentration of 0 or more and 1 ppm or less. More specifically, the gas sensor 10 can detect ozone gas that has an extremely low concentration of 0 or more and 100 ppb or less.

### 1-3. Experimental result

### (Experiment 1)

Next, Experiment 1 for evaluating the effect of the gas sensor 10 will be described.

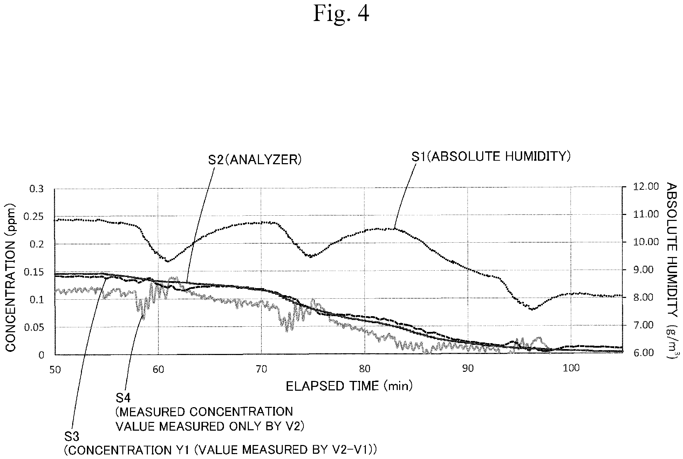

In Experiment 1, the gas sensor 10 having the same configuration as illustrated in Fig. 1 and Fig. 2 was prepared, and the element portion 20 of the gas sensor 10 was disposed in a room in which a humidity and a temperature were adjusted by an air conditioner. In Experiment 1, the ozone gas concentration Y1 was detected by the gas sensor 10 in the method described above in the first embodiment. In Fig. 4, the detection result of the concentration Y1 in Experiment 1 is represented by broken lines S3. In Fig. 4, the horizontal axis represents elapsed time (min (minutes)), and the vertical axis (first axis) represents the concentration (ppm) .

In Experiment 1, for comparison with the detection result of the concentration Y1, an ozone gas concentration in a target space (room that was a space in which the concentration Y1 was detected) was detected by an analyzer (EBARA CORPORATION EG3000F) in parallel with the detection of the concentration Y1. The result of detecting the ozone gas concentration in the target space by the analyzer is represented by a thick line S2 in Fig. 4. The detection result represented by the thick line S2 is represented according to the first axis on the left side and the horizontal axis.

In Experiment 1, in order to evaluate influence of an absolute humidity (that is, influence of an amount of water vapor in the space), an absolute humidity in the target space was detected in parallel with the detection of the concentration Y1. The result of detecting an absolute humidity in the target space is represented by a dotted line S1 in Fig. 4. The detection result represented by the dotted line S1 is represented according to a second axis on the right side and the horizontal axis. The second axis represents an absolute humidity (g/m3).

In Experiment 1, in order to evaluate a result of measurement in which the voltage V1 was not used (in which influence of water vapor was not canceled), a result of measurement in which an ozone gas concentration in the target space was detected only by the voltage V2 (in which the ozone gas concentration was detected only by the second sensing element 22) was obtained in parallel with the detection of the concentration Y1. The result of the measurement in which the ozone gas concentration was detected only by the voltage V2 is represented by a solid line S4 in Fig. 4. The detection result represented by the solid line S4 is represented according to the first axis on the left side and the horizontal axis.

According to the experimental result of Experiment 1, in a case where the ozone gas concentration was detected only by the voltage V2, influence of change of an absolute humidity (that is, influence of change in an amount of water vapor) became great. Particularly in a case where an absolute humidity greatly changed, the detection result of the ozone gas concentration greatly deviated from the detection result of the ozone gas concentration obtained by the analyzer. Meanwhile, in a case where the concentration Y1 was detected by the gas sensor 10, even when an absolute humidity greatly changed, the concentration Y1 did not greatly deviate from the ozone gas concentration detected by the analyzer. Thus, it can be seen that influence of an absolute humidity was reduced.

### (Experiment 2)

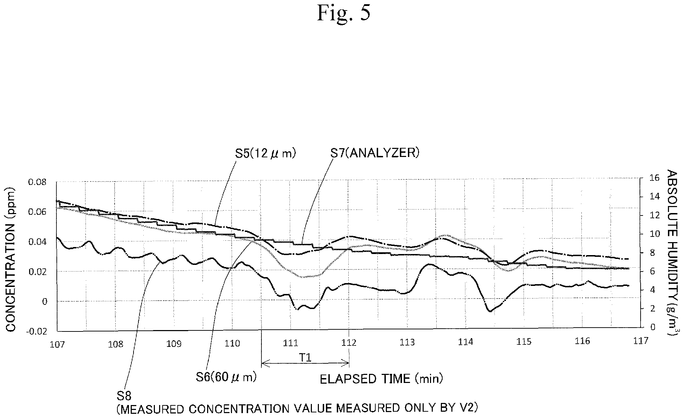

In Experiment 2, two kinds of the gas sensors 10 each having the same configuration as illustrated in Fig. 1 and Fig. 2 were prepared. The two kinds of the gas sensors 10 were different from each other only in the thickness of the moisture permeable film 24 (film thickness). The thickness of the moisture permeable film 24 of one of the gas sensors 10 was 60 um. The thickness of the moisture permeable film 24 of the other of the gas sensors 10 was 12 um. In Experiment 2, the element portions 20 of the two kinds of the gas sensors 10 were disposed in a room in which a humidity and a temperature were adjusted by an air conditioner. Each of the two kinds of the gas sensors 10 detected the ozone gas concentration Y1 in the method described above in the first embodiment. The detection result of the concentration Y1 by the gas sensor 10 in which the moisture permeable film 24 had a thickness of 12 um is represented by an alternate long and short dash line S5 in Fig. 5. The detection result of the concentration Y1 by the gas sensor 10 in which the moisture permeable film 24 had a thickness of 60 um is represented by a solid line S6 in Fig. 5. In Fig. 5, the horizontal axis represents elapsed time (min (minutes)) and the vertical axis represents the concentration (ppm).

In Experiment 2, for comparison with the detection result of the concentration Y1 by each of the gas sensors 10, an ozone gas concentration in a target space (room that was a space in which the concentration Y1 was detected) was detected by an analyzer (EBARA CORPORATION EG2000) in parallel with the detection by each of the gas sensors 10. The result of detecting the ozone gas concentration in the target space by the analyzer is represented by a thick line S7 in Fig. 5.

In Experiment 2, in order to evaluate a result of measurement in which the voltage V1 was not used (in which influence of water vapor was not canceled), a result of measurement in which an ozone gas concentration in the target space was detected only by the voltage V2 (in which the ozone gas concentration was detected only by the second sensing element 22) was obtained in parallel with the detection of the concentration Y1. The result of the measurement in which the ozone gas concentration was detected only by the voltage V2 is represented by an alternate long and two short dashes line S8 in Fig. 5.

As indicated in a period T1 in Fig. 5, in a case where the measurement result represented by the alternate long and two short dashes line S8 (result of measurement in which influence of water vapor was not cancelled) greatly changed even though the ozone gas concentration detected by the analyzer did not greatly change, the period T1 may be considered to be a time period in which an absolute humidity rapidly changed without great change of the ozone gas concentration (that is, time period in which an amount of water vapor in the space rapidly changed). According to the experimental result of Experiment 2, in such a time period in which the absolute humidity rapidly changed, deviation of the detection result represented by the alternate long and short dash line S5 from the detection result represented by the thick line S7 (detection result by the analyzer) is smaller than deviation of the detection result represented by the solid line S6 from the detection result represented by the thick line S7. That is, the gas sensor 10 in which the thickness of the moisture permeable film 24 was relatively small was able to cancel influence of an absolute humidity with better responsiveness even when the absolute humidity rapidly changed.

### 1-4. Example of effect of gas sensor

In the electrochemical gas sensor, when there is a difference in an amount of water vapor between inside and outside the element, an electromotive force caused by the difference in the amount of water vapor may be generated, and an electric current caused by the difference in the amount of water vapor may flow. In the case of a low-concentration target gas in which a concentration of a to-be-detected gas (for example, ozone gas) is 1 ppm or less, influence of an electric current caused by difference in an amount of water vapor tends to be great when detecting the concentration of the to-be-detected gas. Regarding this, in the gas sensor 10, since the first sensing element 21 detects a concentration of a to-be-detected gas in a state in which the to-be-detected gas is substantially removed from a target gas, influence of an electric current caused by difference in an amount of water vapor can be evaluated. Meanwhile, the second sensing element 22 can detect a concentration of a target component containing water vapor and the to-be-detected gas. That is, the gas sensor 10 can use the detection result of the first sensing element 21 (result of evaluating influence of an electric current caused by difference in an amount of water vapor) for detecting a concentration of the to-be-detected gas by the second sensing element 22, which is advantageous in correctly detecting a concentration of a to-be-detected gas in a target gas in which the concentration of the to-be-detected gas is low. Therefore, even when a to-be-detected gas contained in a target gas has a low concentration of 1 ppm or less, the gas sensor 10 can more correctly detect a concentration of the to-be-detected gas.

Furthermore, as in the gas sensor 10, when the moisture permeable film 24 is structured to have a thickness of 60 um or less, degradation of responsiveness with respect to change of a humidity can be further inhibited in the detection by the first sensing element.

The gas sensor 10 is, more specifically, capable of detecting the to-be-detected gas having a concentration of 0 or more and 100 ppb or less. In the case of an extremely-low-concentration target gas in which a concentration of the to-be-detected gas is 100 ppb or less, influence of an "electric current caused by difference in an amount of water vapor" tends to be much greater when detecting the concentration of the to-be-detected gas. Regarding this, the gas sensor 10 can correctly evaluate influence of an "electric current caused by difference in an amount of water vapor" and detect a concentration of the to-be-detected gas by reflecting the evaluation result, so that the gas sensor 10 is very useful in a case where a target is gas in which the concentration of the to-be-detected gas is 100 ppb or less.

The gas sensor 10 includes the controller 14 corresponding to an example of a concentration specifying portion, and can specify a concentration of the to-be-detected gas (ozone gas) based on outputs of the first sensing element 21 and the second sensing element 22, and the sensitivity β. Furthermore, the gas sensor 10 can adjust the sensitivity β based on an actually measured humidity of a target gas. Therefore, the sensitivity β can be made appropriate according to a humidity of a target gas obtained during detection.

In the gas sensor 10, the to-be-detected gas is ozone (ozone gas). When a concentration of ozone is detected by the gas sensor 10, water vapor may become noise, and the ozone concentration may be hindered from being correctly detected. Particularly, when a target is gas in which a concentration of ozone is low, influence of an "electric current caused by difference in an amount of water vapor" becomes relatively great, so that such a problem becomes more significant. However, the gas sensor 10 can correctly evaluate influence of an "electric current caused by difference in an amount of water vapor", and can use the evaluation for detecting a concentration of ozone.
Therefore, the gas sensor 10 is extremely advantageous in correctly detecting an ozone concentration in a target gas in which an ozone concentration is low.

### 1-5. Ozone generator

Fig. 6 illustrates an ozone generator 100 including the gas sensor 10 described above. The ozone generator 100 has a humidifying function. The ozone generator 100 corresponds to an example of a humidifier, and has a function of humidifying the space 90 as the external space.

The ozone generator 100 has the gas sensor 10, an ozone generation device 102, a fan 104, a humidifying device 112, a fan 114, and a case 120. The gas sensor 10 incorporated in the ozone generator 100 has a communication unit 17, a display unit 18, a storage unit 19, and the like as well as the sensing portion 12 and the controller 14 described above.

The ozone generator 100 is configured such that an opening 122 is formed in the case 120, and gas outside the ozone generator 100 can be introduced into the sensing portion 12 through the opening 122. That is, a space outside the ozone generator 100 and the space 92 in the element portion 20 are connected with each other, and gas can mutually flow into and out of the spaces.

The communication unit 17 is a unit for performing wired communication or wireless communication with an external device disposed outside the gas sensor 10. The communication unit 17 may be configured to communicate with a device in the ozone generator 100 or may be configured to communicate with a device outside the ozone generator 100.

The display unit 18 is a unit for displaying symbols such as characters and numerals, and images such as pictures. The display unit 18 can display various information in cooperation with the controller 14. The storage unit 19 is a device for storing various information.

The ozone generation device 102 is a device for generating ozone gas. Various known methods can be adopted as a method by which the ozone generation device 102 generates ozone (ozone gas). For example, a known method such as an ultraviolet method, an electrolysis method, or an electric discharge method can be adopted. The ozone generation device 102 feeds ozone gas generated by itself to a supply tube 106.

The supply tube 106 is a tube for guiding ozone gas generated by the ozone generation device 102 to a space outside the ozone generator 100. An amount and a flow rate of gas flowing through the supply tube 106 are adjusted by the fan 104. The fan 104 is configured as a blower (air sending machine). The fan 104 has, for example, a rotor (not illustrated) having a plurality of blades, a power unit (not-illustrated motor or the like) for rotating the rotor, and a drive circuit (not illustrated) for adjusting the rotation of the power unit. The drive circuit controls the number of revolutions of the rotor such that the number of revolutions of the rotor becomes the number of revolutions instructed by the controller 14. The greater the number of revolutions of the rotor of the fan 104 is, the greater an amount and a flow rate of gas flowing through the supply tube 106 are.

In a case where the ozone generation device 102 generates ozone gas at a constant generation rate, the greater the number of revolutions of the fan 104 becomes, the greater an amount and a flow rate of ozone gas flowing through the supply tube 106 become. Therefore, the controller 14 can adjust a rate at which ozone gas is supplied (an amount of ozone gas supplied into a space per unit time) by adjusting the number of revolutions of the fan 104.

The humidifying device 112 is a device for generating water vapor or mist. Various known methods can be adopted as a method by which the humidifying device 112 generates water vapor or mist. For example, a known method such as a steam method, an ultrasonic method, a vaporization method, and a hybrid method can be adopted. The humidifying device 112 feeds water vapor or mist generated by itself to a supply tube 116.

The supply tube 116 is a tube for guiding water vapor or mist generated by the humidifying device 112 to a space outside the ozone generator 100. An amount and a flow rate of gas flowing through the supply tube 116 are adjusted by the fan 114. The fan 114 is configured as a blower (air sending machine). The fan 114 has, for example, a rotor (not illustrated) having a plurality of blades, a power unit (not-illustrated motor or the like) for rotating the rotor, and a drive circuit (not illustrated) for adjusting the rotation of the power unit. The drive circuit controls the number of revolutions of the rotor such that the number of revolutions of the rotor becomes the number of revolutions instructed by the controller 14. The greater the number of revolutions of the rotor of the fan 114 is, the greater an amount and a flow rate of gas flowing through the supply tube 116 are.

In a case where the humidifying device 112 generates water vapor or mist at a constant generation rate, the greater the number of revolutions of the fan 114 becomes, the greater an amount and a flow rate of water vapor or mist flowing through the supply tube 116 become. Therefore, the controller 14 can adjust a rate at which water vapor or mist is supplied (an amount of water vapor or mist supplied into a space per unit time) by adjusting the number of revolutions of the fan 114.

In the ozone generator 100, the ozone gas concentration Y1 detected by the gas sensor 10 in the above-described method can be utilized. For example, the controller 14 may display on the display unit 18 the detected ozone gas concentration Y1 (ozone gas concentration in a space in which the ozone generator 100 is disposed) as numerals, in cooperation with the display unit 18. The controller 14 may display on the display unit 18 an absolute humidity detected by the humidity sensor 15 (absolute humidity in a space in which the ozone generator 100 is disposed) as numerals. In a case where the humidity sensor 15 functions as a relative temperature sensor, the controller 14 may display on the display unit 18 a relative humidity specified by the humidity sensor 15 (relative humidity in a space in which the ozone generator 100 is disposed).

The controller 14 may transmit the detected ozone gas concentration Y1 to an external device disposed outside the ozone generator 100 in cooperation with the communication unit 17. Alternatively, the controller 14 may transmit the above-described absolute humidity or relative humidity to an external device disposed outside the ozone generator 100 in cooperation with the communication unit 17.

In addition to merely discharging ozone into a space, the ozone generator 100 can actually measure an ozone concentration in the space accurately, and use the measured ozone concentration to enhance convenience for a user.

In a case where the ozone generator 100 is regarded as a humidifier, the humidifier can not only merely humidify a space but also actually measure an ozone concentration in the space accurately, and use the measured ozone concentration to enhance convenience for a user.

### 2. Second embodiment

### 2-1. Configuration of ozone generator

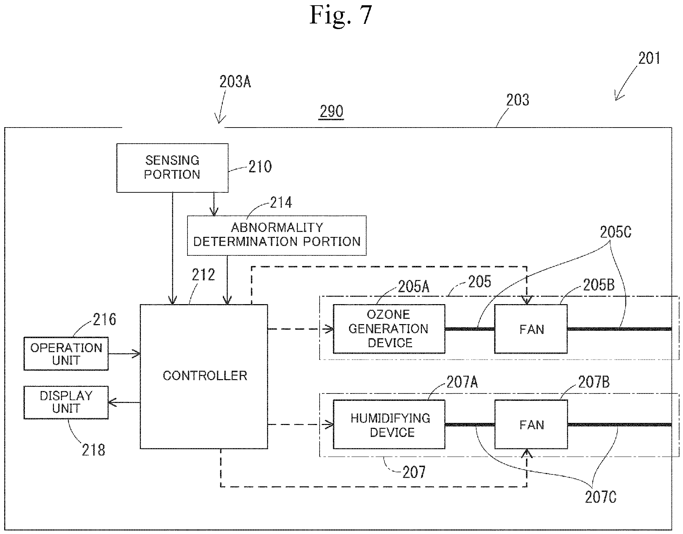

Fig. 7 illustrates an ozone generator 201 according to a second embodiment. The ozone generator 201 illustrated in Fig. 7 is a device for generating ozone and supplying the ozone to an external space 290 outside the ozone generator 201. The ozone generator 201 includes a case 203, an ozone supply portion 205, a humidifying portion 207, a sensing portion 210, a controller 212, an abnormality determination portion 214, an operation unit 216, and a display unit 218.

In the case 203, the ozone supply portion 205, the humidifying portion 207, the sensing portion 210, the controller 212, the abnormality determination portion 214, the operation unit 216, and the display unit 218 are stored. A space outside the case 203 is a space outside the ozone generator 201, and is the external space 290. The case 203 has an opening 203A. Gas in the external space 290 can be introduced into the sensing portion 210 through the opening 203A.

The ozone supply portion 205 generates ozone and supplies the generated ozone to the external space 290. The ozone supply portion 205 has an ozone generation device 205A, a fan 205B, and a supply tube 205C. The ozone generation device 205A is a device for generating ozone. A method by which the ozone generation device 205A generates ozone is not limited. In the present embodiment, the ozone generation device 205A has an AC power supply (not illustrated) such as a transformer, and an ozone generation unit (not illustrated). The AC power supply converts DC voltage to AC voltage. The ozone generation unit performs discharging (for example, dielectric barrier discharge) by being applied with AC voltage obtained through conversion by the AC power supply, and generates ozone by using, as a material, oxygen in a target gas (for example, air) introduced from the external space 290. An amount of ozone generated by the ozone generation device 205A is adjusted by adjusting voltage applied to the ozone generation device 205A (more specifically, an effective value of AC voltage applied to the ozone generation unit). The higher the voltage applied to the ozone generation unit (more specifically, an effective value of AC voltage applied to the ozone generation unit) is, the greater an amount of generated ozone is. The ozone generation device 205A feeds the ozone generated by itself to the supply tube 205C.

The supply tube 205C is a tube for guiding ozone generated by the ozone generation device 205A to the external space 290. An amount and a flow rate of gas flowing through the supply tube 205C are adjusted by the fan 205B.

The fan 205B is configured as a blower (air sending machine). The fan 205B has, for example, a rotor (not illustrated) having a plurality of blades, a power unit (not-illustrated motor or the like) for rotating the rotor, and a drive circuit (not illustrated) for adjusting the rotation of the power unit. The drive circuit controls the number of revolutions of the rotor such that the number of revolutions of the rotor becomes the number of revolutions instructed by the controller 212. The greater the number of revolutions of the rotor of the fan 205B is, the greater an amount and a flow rate of gas flowing through the supply tube 205C are.

An amount of ozone supplied by the ozone supply portion 205 to the external space 290 is adjusted by adjusting at least one of voltage applied to the ozone generation device 205A and the number of revolutions of the fan 205B.

The humidifying portion 207 supplies water vapor or mist to the external space 290 outside the ozone generator 201 and humidifies the external space 290. The humidifying portion 207 has a humidifying device 207A, a fan 207B, and a supply tube 207C.

The humidifying device 207A is a device for generating water vapor or mist. Various known methods can be adopted as a method by which the humidifying device 207A generates water vapor or mist. For example, a known method such as a steam method, an ultrasonic method, a vaporization method, and a hybrid method can be adopted. The humidifying device 207A feeds water vapor or mist generated by itself to the supply tube 207C.

The supply tube 207C is a tube for guiding water vapor or mist generated by the humidifying device 207A to the external space 290 outside the ozone generator 201. An amount and a flow rate of gas flowing through the supply tube 207C are adjusted by the fan 207B.

The fan 207B is configured as a blower (air sending machine). The fan 207B has, for example, a rotor (not illustrated) having a plurality of blades, a power unit (not-illustrated motor or the like) for rotating the rotor, and a drive circuit (not illustrated) for adjusting the rotation of the power unit. The drive circuit controls the number of revolutions of the rotor such that the number of revolutions of the rotor becomes the number of revolutions instructed by the controller 212. The greater the number of revolutions of the rotor of the fan 207B is, the greater an amount and a flow rate of gas flowing through the supply tube 207C are.

In a case where the humidifying device 207A generates water vapor or mist at a constant generation rate, the greater the number of revolutions of the fan 207B becomes, the greater an amount and a flow rate of water vapor or mist flowing through the supply tube 207C become. Therefore, the controller 212 can adjust a rate at which water vapor or mist is supplied (an amount of water vapor or mist supplied to the external space 290 per unit time) by adjusting the number of revolutions of the fan 207B.

The sensing portion 210 is an ozone sensor for electrochemically detecting an ozone concentration. The "electrochemically" means, for example, a potentiostatic electrolysis method. The sensing portion 210 detects an ozone concentration in air introduced from the external space 290. A signal indicating an ozone concentration detected by the sensing portion 210 (hereinafter, also referred to as "detected concentration") is inputted to each of the controller 212 and the abnormality determination portion 214. The configuration of the sensing portion 210 may be the same as the configuration of the sensing portion 12 of the first embodiment, or may be different from the configuration of the sensing portion 12. In a typical example, the sensing portion 210 has the same configuration as the sensing portion 12, and has the first sensing element 21, the second sensing element 22, and the like.

Each of the controller 212 and the abnormality determination portion 214 is, for example, composed of a microcomputer as a main unit, and has a CPU, a ROM, a RAM, and the like. The controller 212 controls operations of the ozone supply portion 205, the humidifying portion 207, and the display unit 218. The operation unit 216 is, for example, a push switch. The display unit 218 is, for example, a LED lamp, a liquid crystal display, or the like.

### 2-2. Operation of ozone generator

The controller 212 performs first control for performing feedback control for the ozone supply portion 205 such that an ozone concentration detected by the sensing portion 210 approaches a target concentration.

The target concentration may be a predetermined fixed value, or may be a value that is set through a setting operation performed by using the operation unit 216. The target concentration is preferably high from the viewpoint of inactivating virus floating in the external space 290, but is preferably set so as not to adversely affect human bodies. For example, the target concentration is preferably more than 0 ppb (parts per billion) and 100 ppb or less. In the present embodiment, the target concentration is 50 ppb.

The controller 212 performs feedback control for the ozone supply portion 205 in the first control by adjusting for example, voltage to be applied to the ozone generation device 205A of the ozone supply portion 205. In the present embodiment, in the first control performed by the controller 212, the number of revolutions of the rotor of the fan 205B is constant. However, the number of revolutions of the rotor of the fan 205B may vary.

In a case where a determination criterion is satisfied during the first control, the controller 212 performs second control for stopping the first control. The determination criterion is not particularly limited. The determination criterion may be, for example, a condition in which a second predetermined period (for example, 120 minutes) has elapsed from the start of the first control. The second predetermined period may be a predetermined fixed value, may vary according to an ozone concentration or the like, or may be a value that is set through a setting operation performed by using the operation unit 216. The second predetermined period may be determined based on an assumed upper limit value of an ozone concentration during the first control. For example, in a case where the upper limit value is assumed to be 160 ppb, the second predetermined period may be shorter than 200 minutes (for example, 120 minutes) by which human bodies are not affected even if the state of 160 ppb continues. Another example of the determination criterion may be a condition in which a determination operation has been performed by the operation unit 216.

The second control is, for example, control for supplying a constant amount of ozone, control for supplying ozone with regular change, or control for stopping the ozone supply portion 205. In the second control, for example, the controller 212 controls the ozone supply portion 205 so as to supply low-concentration ozone, or stops the ozone supply portion 205. That is, in the second control, for example, the controller 212 controls the ozone supply portion 205 so as to supply a constant amount of low-concentration ozone, controls the ozone supply portion 205 so as to supply low-concentration ozone with regular change, or stops the ozone supply portion 205.

The "low concentration" means that an amount is less than an amount of ozone supplied from the ozone supply portion 205 when the determination criterion is satisfied during the first control, and may be a "predetermined value" or may be a "value determined based on a detected concentration". In the present embodiment, it is assumed that gas other than ozone affects in a plus side with respect to a detected concentration. Therefore, the "predetermined value" may be, for example, a value less than an ozone supply amount required to maintain an actual concentration at a target concentration in an environment where there is no influence of gas other than ozone. The "value determined based on a detected concentration" may be, for example, 30% or less of an amount of ozone that is supplied from the ozone supply portion 205 when the determination criterion is satisfied during the first control. In a case where the rotation speed of the rotor of the fan 205B is constant, an amount of ozone supplied from the ozone supply portion 205 has a correlation with voltage applied to the ozone supply portion 205, and thus can be compared with voltage that is applied to the ozone supply portion 205 when the determination criterion is satisfied during the first control. That is, while maintaining the rotation speed of the rotor of the fan 205B at a rotation speed at the time when the determination criterion is satisfied, the controller 212 can set an amount of ozone supplied from the ozone supply portion 205 to 30% or less by setting the voltage applied to the ozone supply portion 205 to be 30% or less of the voltage that is applied to the ozone supply portion 205 when the determination criterion is satisfied during the first control.

The abnormality determination portion 214 determines abnormality based on a degree of change from satisfaction of the determination criterion of the ozone concentration detected by the sensing portion 210 until elapse of a predetermined period. In the description herein, the "abnormality" means "abnormality in which an actual ozone concentration during feedback control deviates from a target concentration". The sensing portion 210 which electrochemically detects an ozone concentration as described above is also influenced by gas other than ozone. For example, in a case where there is a reducing gas having properties opposite to properties of ozone which is an oxidizing gas, it is assumed that the reducing gas affects in the minus direction with respect to the detection result by the sensing portion 210 which electrochemically detects an ozone concentration. In this case, the detected concentration is lower than the actual ozone concentration (hereinafter, also referred to as "actual concentration"). Thus, when the controller 212 performs feedback control in the first control such that the detected concentration approaches the target concentration, the actual concentration becomes higher than the target concentration. The abnormality determination portion 214 can determine abnormality that occurs in this way. The predetermined period is a period determined in advance and is, for example, 10 minutes.

The abnormality determination portion 214 determines abnormality based on the degree of reduction from satisfaction of the determination criterion of the detected concentration until elapse of the predetermined period, in the configuration where the controller 212 controls the ozone supply portion 205 so as to supply low-concentration ozone or stops the ozone supply portion 205 in the second control. When the controller 212 controls the ozone supply portion 205 so as to supply low-concentration ozone or stops the ozone supply portion 205 in the second control, the detected concentration decreases during the second control. At this time, the higher the actual concentration is, the greater the degree of reduction of the detected concentration tends to be. Therefore, the abnormality determination portion 214 can determine abnormality based on the degree of reduction of the detected concentration.

The abnormality determination portion 214 determines abnormality in a case where the degree of reduction is determined to be greater than a threshold value. As described above, in a case where there is a reducing gas, the actual concentration tends to be higher than the target concentration in the first control. The higher the actual concentration is, the greater the degree of reduction when the second control is performed is. Therefore, in a case where there is a reducing gas, the degree of reduction becomes great when the second control is performed, as compared with a case where there is no reducing gas (that is, in a case where the actual concentration is equal to the target concentration).

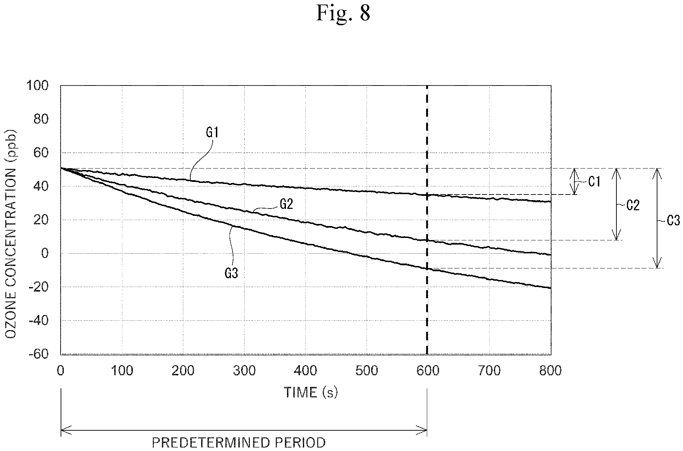

The abnormality determination portion 214 previously grasps a degree of reduction when the predetermined period elapses in a case where an actual concentration when the determination criterion is satisfied is an abnormal value, and thereby can determine abnormality. Fig. 8 illustrates graphs G1, G2, G3 based on actually measured values. G1 represents an ozone concentration changing with elapse of time when the second control (in this case, stop of the ozone supply portion 205) is performed in a state where the actual concentration is equal to a target concentration (50 ppb). G2 represents an ozone concentration changing with elapse of time when the second control is performed in a state where an actual concentration is 100 ppb. G3 represents an ozone concentration changing with elapse of time when the second control is performed in a state where an actual concentration is 130 ppb. C1 represents a degree of reduction from start of the second control until elapse of the predetermined period in a state where the actual concentration is equal to the target concentration. C2 represents a degree of reduction from start of the second control until elapse of the predetermined period in a state where the actual concentration is 100 ppb. C3 represents a degree of reduction from start of the second control until elapse of the predetermined period in a state where the actual concentration is 130 ppb.

The above-described "threshold value" is greater than a value of C1, and is C2 in the present embodiment. In a case where an actual concentration when the determination criterion is satisfied is higher than 100 ppb as indicated by G3 in Fig. 8, the degree of reduction is greater than C2 which serves as the threshold value. Therefore, the abnormality determination portion 214 determines that the degree of reduction is greater than the threshold value, and determines that it is abnormal. Meanwhile, in a case where an actual concentration when the determination criterion is satisfied is not higher than 100 ppb as indicated by G1 in Fig. 8, the degree of reduction is not greater than C2 which serves as the threshold value. Therefore, the abnormality determination portion 214 determines that the degree of reduction is not greater than the threshold value, and does not determine that it is abnormal.

The controller 212 performs the first control in a case where the degree of reduction is determined to be not greater than the threshold value, and, the controller 212 performs third control in a case where the degree of reduction is determined to be greater than the threshold value. The third control is a control for performing feedback control for the ozone supply portion 205 such that the detected concentration approaches a second target concentration lower than the target concentration, or causing the ozone supply portion 205 to supply low-concentration ozone, or stopping the ozone supply portion 205.

The third control may be control different from the second control, or may be the same control as the second control. That is, the controller 212 may change or may not change contents of the control when shifting from the second control to the third control. As an example in which the third control is different from the second control, the ozone supply portion 205 may be stopped in the second control, and the ozone supply portion 205 may be caused to supply low-concentration ozone in the third control. As an example in which the third control is the same control as the second control, the ozone supply portion 205 may be controlled so as to supply low-concentration ozone in the second control, and the ozone supply portion 205 may be maintained so as to supply low-concentration ozone in the third control.

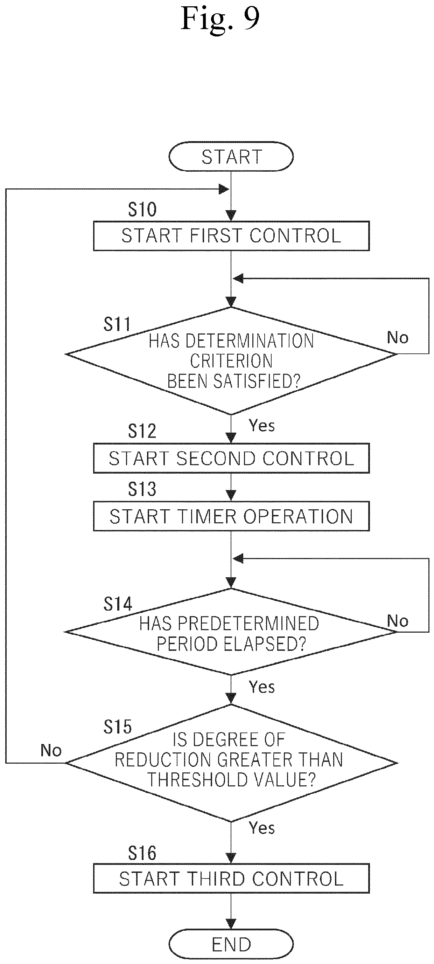

When a start criterion is satisfied, the controller 212 and the abnormality determination portion 214 of the ozone generator 201 perform the process illustrated in Fig. 9 in cooperation. The start criterion is, for example, that a starting operation is performed by the operation unit 216.

When the start criterion is satisfied, the controller 212 starts the above-described first control in step S10. After the first control has been started, the controller 212 determines in step S11 whether or not the above-described determination criterion has been satisfied. In a case where the controller 212 determines that the determination criterion is not satisfied (in the case of No in step S11), the process is returned to step S11. That is, the controller 212 repeatedly determines whether or not the determination criterion is satisfied until the determination criterion is satisfied.

In a case where the controller 212 determines that the determination criterion has been satisfied (in the case of Yes in step S11), the controller 212 stops the first control and starts the second control in step S12. The abnormality determination portion 214 starts an operation of a timer in step S13. The abnormality determination portion 214 determines in step S14 whether or not a predetermined period has elapsed based on the timer operated in step S13. In a case where the abnormality determination portion 214 determines that the predetermined period has not elapsed (in the case of No in step S14), the process is returned to step S14. That is, the abnormality determination portion 214 repeatedly determines whether or not the predetermined period has elapsed until the predetermined period has elapsed.

In a case where the abnormality determination portion 214 determines that the predetermined period has elapsed (in the case of Yes in step S14), the abnormality determination portion 214 determines in step S15 whether or not the degree of reduction of the detected concentration is greater than a threshold value. In a case where the abnormality determination portion 214 determines that the degree of reduction of the detected concentration is not greater than the threshold value (in the case of No in step S15), the controller 212 returns the process to step S10 to stop the second control and start the first control. In a case where the abnormality determination portion 214 determines that the degree of reduction of the detected concentration is greater than the threshold value (in the case of Yes in step S15), the controller 212 stops the second control and starts the third control in step S16. That is, in a case where the determination criterion is satisfied during the first control, the controller 212 performs the second control. Then, in a case where the degree of reduction of the detected concentration is not greater than the threshold value, the controller 212 restarts the first control, and, in a case where the degree of reduction of the detected concentration is greater than the threshold value, the controller 212 causes the ozone supply portion 205 so as to supply low-concentration ozone or stops the ozone supply portion 205.

### 2-3. Effect of ozone generator of second embodiment

Next, the effect of the ozone generator 201 of the second embodiment will be described.

The sensing portion 210 electrochemically detects an ozone concentration, and may thus be influenced by gas (for example, cigarette smoke or incense) other than ozone. As a result, the detected concentration may deviate from an actual concentration. If feedback control is performed for the ozone supply portion 205 in this state such that the detected concentration approaches a target concentration, the actual concentration deviates from the target concentration. In order to determine such abnormality, the ozone generator 201 performs the second control for stopping the first control in a case where the determination criterion is satisfied during the first control. The ozone generator 201 determines abnormality based on the degree of change of the detected concentration from satisfaction of the determination criterion until elapse of a predetermined period. The sensing portion 210 which electrochemically detects an ozone concentration has characteristics that, the higher the actual concentration is, the more the detected concentration tends to decrease. Therefore, when the first control has been stopped, a degree of change of the detected concentration varies according to the actual concentration. Based on the degree of the change, the ozone generator 201 can determine abnormality in which the actual concentration during feedback control deviates from a target concentration.

Furthermore, in the second control, the ozone generator 201 controls the ozone supply portion 205 so as to supply low-concentration ozone or stops the ozone supply portion 205, and determines abnormality based on the degree of reduction of the detected concentration from satisfaction of the determination criterion until elapse of a predetermined period. Therefore, the ozone generator 201 can determine abnormality while inhibiting increase of the ozone concentration in the external space 290.

Furthermore, the ozone generator 201 determines abnormality in a case where the degree of reduction is determined to be greater than a threshold value. Therefore, the ozone generator 201 can determine abnormality in which an actual ozone concentration during feedback control becomes higher than a target concentration.

Furthermore, in a case where the degree of reduction is determined to be not greater than a threshold value, the ozone generator 201 restarts the first control, and, in a case where the degree of reduction is determined to be greater than the threshold value, the ozone generator 201 can reduce or stop supply of ozone.

Furthermore, the ozone generator 201 includes the humidifying portion 207. Therefore, the ozone generator 201 can also function as a humidifier.

### <Other embodiments>

The present invention is not limited to the embodiments described above with reference to the drawings, and, for example, the following embodiments are also included in the technical scope of the present invention. Various characteristics of the embodiments described above and embodiments described below may be combined in any way as long as there is no contradiction.

In the above-described first embodiment, the gas sensor 10 in which a to-be-detected gas is ozone gas is described. However, the to-be-detected gas of the gas sensor 10 may be gas other than ozone gas. For example, ethylene gas may be the to-be-detected gas. In this example, the gas sensor 10 may function as an ethylene gas sensor.

In the above-described first embodiment, the ozone generation device 102 and the humidifying device 112 are configured as separate devices. However, the ozone generation device 102 and the humidifying device 112 may be configured integrally. That is, the ozone generation device 102 may have a humidifying function of discharging water vapor or mist. In a case where the ozone generation device 102 thus has a humidifying function, the fan 114 may be omitted, and the fan 104 may be used to adjust a supply rate of ozone and a supply rate of water vapor or mist.

In the above-described first embodiment, as an example of a method for adjusting a supply rate of ozone gas, an example in which a flow rate is adjusted by the fan has been described. However, the present invention is not limited to this example. For example, in the ozone generator 100, instead of using the fan 104, or using the fan 104 in combination, a method in which a flow rate is adjusted by a flow rate regulating valve may be adopted, or, an ozone supply state and an ozone supply stop state may be switched simply by opening/closing a flow path by an on-off valve. Alternatively, the ozone generator 100 may adjust supply of ozone by switching between a state in which the ozone generation device 102 is operated and a state in which the operation is stopped, without providing the fan 104 or using the fan 104 in combination.

In the above-described first embodiment, as an example of the method for adjusting a supply rate of water vapor or mist, an example in which a flow rate is adjusted by the fan has been described. However, the present invention is not limited to this example. For example, in the ozone generator 100, instead of using the fan 114, or using the fan 114 in combination, a method in which a flow rate is adjusted by a flow rate regulating valve may be adopted, or, a state in which water vapor or mist is supplied and a state in which the supply is stopped may be switched simply by opening/closing a flow path by an on-off valve. Alternatively, the ozone generator 100 may adjust supply of water vapor or mist by switching between a state in which the humidifying device 112 is operated and a state in which the operation is stopped, without providing the fan 114 or using the fan 114 in combination.

In the above-described first embodiment, the ozone generator 100 having a humidifying function is described as an example of an ozone generator. However, the present invention is not limited to this example. For example, an ozone generator having no humidifying function may include the gas sensor 10. One of the examples is a configuration in which the humidifying device 112 and the fan 114 are omitted from the configuration illustrated in Fig. 6. In the ozone generator including the gas sensor 10, the configuration other than the gas sensor 10 may be other than the configuration already illustrated. For example, the ozone generation device 102 and the fan 104 may have the same configuration as any of ozone supply portions in various known ozone generators.

In the above-described first embodiment, the ozone generator 100 is described as an example of a humidifier. However, the present invention is not limited to this example. For example, a humidifier having no ozone generating function may include the gas sensor 10. One of the examples is a configuration in which the ozone generation device 102 and the fan 104 are omitted from the configuration illustrated in Fig. 6. In the humidifier including the gas sensor 10, the configuration other than the gas sensor 10 may be a configuration other than the configuration already illustrated. For example, the humidifying device 112 and the fan 114 may have the same configuration as any of humidifying portions in various known humidifiers.

In the above-described first embodiment, the element portion 20 includes the case 30. However, the case 30 may not necessarily be provided.

In the above-described first embodiment, in the element portion 20, the first storage portion 31 and the second storage portion 32 are fixed in the case 30 and integrally configured. However, the first storage portion 31 and the second storage portion 32 may not necessarily be integrally configured and may be separately provided.

In the above-described second embodiment, the ozone generator is configured to determine abnormality in a case where the degree of reduction of the detected concentration is determined to be greater than a threshold value. However, the abnormality may be determined by another method. For example, abnormality may be determined in a case where it is determined that the degree of reduction of the detected concentration does not exceed a threshold value. Gas other than ozone gas may include not only gas that exerts an influence on the detected concentration in the minus direction but also gas that exerts an influence in the plus direction. In a case where there is gas that exerts an influence in the plus direction, an actual concentration is lower than the detected concentration, and accordingly, the degree of reduction is small. As a method for determining such abnormality, it is conceivable to employ a configuration in which abnormality is determined in a case where it is determined that the degree of reduction of the detected concentration does not exceed a threshold value.

In the above-described second embodiment, in a case where the degree of reduction is greater than a threshold value, the ozone generator is configured to perform the same control in the third control regardless of the exceeding level at which the degree of reduction exceeds the threshold value. However, the ozone generator may be configured to perform different control according to the exceeding level. For example, when the controller of the ozone generator causes the ozone supply portion so as to supply low-concentration ozone in the third control, the controller may control the ozone supply portion such that the greater the degree of reduction is, the less a supply amount of ozone is. As the method for determining a supply amount of ozone, for example, correspondence data indicating correspondence relationship between the degree of reduction and a supply amount of ozone which is set to be reduced according to increase of the degree of reduction, may be stored in advance, and the supply amount of ozone may be determined based on the correspondence data and an actual degree of reduction. The correspondence data may be a table or an arithmetic expression.

In the above-described second embodiment, the ozone supply portion and the humidifying portion are configured as separate portions. However, the ozone supply portion may be configured to include the humidifying portion. For example, the ozone supply portion may be configured to generate ozone water through electrolysis of raw material water and atomize or vaporize the ozone water. In this configuration, humidification can be performed along with supply of ozone. As a configuration for atomizing or vaporizing ozone water, the configuration of the humidifying portion described above in the second embodiment can be adopted. Furthermore, although the ozone generator of the above-described second embodiment includes the humidifying portion, the ozone generator may be configured to include no humidifying portion.

In the above-described second embodiment, in the second control for stopping the first control, the ozone generator controls the ozone supply portion so as to supply low-concentration ozone or stops the ozone supply portion. However, the ozone generator may have a different configuration. For example, the ozone generator may control the ozone supply portion so as to supply ozone having a high concentration that is higher than a target concentration. The "high concentration" means that an amount of supplied ozone is greater than an amount of ozone supplied from the ozone supply portion 205 when the determination criterion is satisfied during the first control.

In the above-described second embodiment, the sensing portion 210 and the controller 212 used in the ozone generator 201 may be configured to include all the characteristics of the electrochemical gas sensor 10 of the above-described first embodiment and function as an electrochemical gas sensor similar to the electrochemical gas sensor 10.

It should be understood that the embodiments disclosed herein are in all aspects illustrative and not restrictive. The scope of the present invention is not limited to the embodiments disclosed herein, and is intended to include all modifications within the scope of the claims or within the scope equivalent to the scope of the claims.

### DESCRIPTION OF REFERENCE NUMERALS

10: electrochemical gas sensor
14: controller (concentration specifying portion, sensitivity setting portion)
15: humidity sensor
21: first sensing element
22: second sensing element
24: moisture permeable film
31: first storage portion
31A: first introduction inlet
100: ozone generator (humidifier)
201: ozone generator (humidifier)
205: ozone supply portion
207: humidifying portion
210: sensing portion
212: controller
214: abnormality determination portion
290: external space

## Claims

1. An electrochemical gas sensor for detecting a concentration of a to-be-detected gas contained in a target gas, the gas sensor comprising:
a first sensing element configured to electrochemically detect a concentration of gas;
a second sensing element disposed in a space into which water vapor and the to-be-detected gas contained in the target gas flow and configured to electrochemically detect a concentration of gas;
a first storage portion having, in its own structure, an internal space in which the first sensing element is stored;
a first introduction inlet provided between the internal space and an external space outside the first storage portion; and
a moisture permeable film disposed in the first introduction inlet and configured to substantially prevent permeation of the to-be-detected gas from the external space to the internal space,
the electrochemical gas sensor being capable of cancelling influence of water vapor based on an output of the first sensing element and an output of the second sensing element and detecting the to-be-detected gas having a concentration of 0 or more and 1 ppm or less.

2. The electrochemical gas sensor according to claim 1, wherein the moisture permeable film is a water vapor permeable filter that allows permeation of water vapor from the external space to the internal space and substantially prevents permeation of the to-be-detected gas from the external space to the internal space.

3. The electrochemical gas sensor according to claim 1 or claim 2, wherein a thickness of the moisture permeable film is more than 0 um and 60 um or less.

4. The electrochemical gas sensor according to any one of claim 1 to claim 3, that is capable of detecting the to-be-detected gas having a concentration of 0 or more and 100 ppb or less.

5. The electrochemical gas sensor according to any one of claim 1 to claim 4, comprising:
a sensitivity setting portion configured to set a sensitivity based on a humidity of the target gas detected by a humidity sensor; and
a concentration specifying portion configured to specify a concentration of the to-be-detected gas based on an output of the first sensing element, an output of the second sensing element, and the sensitivity.

6. The electrochemical gas sensor according to any one of claim 1 to claim 5, wherein the to-be-detected gas is ozone.

7. An ozone generator comprising the electrochemical gas sensor according to claim 6.

8. The ozone generator according to claim 7, comprising:
an ozone supply portion configured to supply ozone to the external space;
a sensing portion configured to electrochemically detect an ozone concentration, the sensing portion having the first sensing element and the second sensing element;
a controller configured to perform first control for performing feedback control for the ozone supply portion such that an ozone concentration detected by the sensing portion approaches a target concentration, and second control for stopping the first control in a case where a determination criterion is satisfied during the first control; and
an abnormality determination portion configured to determine abnormality based on a degree of change from satisfaction of the determination criterion of an ozone concentration detected by the sensing portion until elapse of a predetermined period.

9. The ozone generator according to claim 8, wherein
in the second control, the controller controls the ozone supply portion so as to supply low-concentration ozone or stops the ozone supply portion, and
the abnormality determination portion determines abnormality based on a degree of reduction from satisfaction of the determination criterion of an ozone concentration detected by the sensing portion until elapse of the predetermined period.

10. The ozone generator according to claim 9, wherein the abnormality determination portion determines abnormality in a case where the degree of reduction is determined to be greater than a threshold value.

11. The ozone generator according to claim 10,
wherein the controller performs the first control in a case where the degree of reduction is determined to be not greater than the threshold value, and performs third control in a case where the degree of reduction is determined to be greater than the threshold value, and in the third control, the controller performs feedback control for the ozone supply portion such that a concentration detected by the sensing portion approaches a second target concentration that is lower than the target concentration, or causes the ozone supply portion so as to supply low-concentration ozone, or stops the ozone supply portion.

12. The ozone generator according to claim 11, wherein, when the controller causes the ozone supply portion so as to supply low-concentration ozone in the third control, the controller controls the ozone supply portion such that the greater the degree of reduction is, the less a supply amount of ozone is.

13. A humidifier comprising the electrochemical gas sensor according to any one of claim 1 to claim 7.
